# EUROPEAN PATENT SPECIFICATION

(11) **EP 2 436 315 B1**
(45) Date of publication and mention of the grant of the patent: **04.03.2020**
(21) Application number: 11250838.7
(22) Date of filing: 03.10.2011
(51) Int. Cl.: A61B 17/00

(54) **Specimen retrieval device**
Probenauffindungsvorrichtung
Dispositif de prélèvement de spécimen

(30) Priority: 06.01.2011 US 430206 P; 04.10.2010 US 389391 P; 19.09.2011 US 201113235587
(43) Date of publication of application: 04.04.2012
(73) Proprietor: Covidien LP, Mansfield, MA 02048 (US)
(72) Inventor: Fleming, Alistair Ian, Cambridge CB23 6ER (GB); Grover, Simon Roderick, Cambridge CB1 3EG (GB); Seegert, Charles Alan, Irving, TX 75038 (US)
(74) Representative: Soames, Candida Jane

(56) References cited:
- WO-A2-2004/112571
- WO-A2-2008/114234
- US-A- 5 647 372
- US-A1- 2004 138 587

## Description

### Technical Field

The present disclosure relates to a specimen retrieval device. More particularly, the present disclosure relates to a specimen retrieval device configured for use in minimally invasive surgical procedures.

### Background of Related Art

In minimally invasive surgical procedures, operations are carried out within the body by using elongated instruments inserted through small entrance openings in the body. The initial opening in the body tissue to allow passage of instruments to the interior of the body may be a natural passageway of the body, or it can be created by a tissue piercing instrument such as a trocar, or created by a small incision into which a cannula is inserted.

Because the tubes, instrumentation, and any required punctures or incisions are relatively small, the surgery is less invasive as compared to conventional surgical procedures in which the surgeon is required to cut open large areas of body tissue. Therefore, minimally invasive surgery minimizes trauma to the patient and reduces patient recovery time and hospital costs.

Minimally invasive procedures may be used for partial or total removal of body tissue or organs from the interior of the body, e.g. nephrectomy, cholecystectomy, lobectomy and other procedures including thoracic, laparoscopic and endoscopic procedures. During such procedures, it is common that a cyst, tumor, or other affected tissue or organ needs to be removed via the access opening in the skin, or through a cannula. Various types of entrapment devices have been disclosed to facilitate this procedure. In many procedures where cancerous tumors are removed, removal of the specimen in an enclosed environment is highly desirable to prevent seeding of cancer cells.

In minimally invasive thoracic surgery, access to the thoracic cavity is limited as well as maneuverability within the cavity as the access port is placed between the confined space between a patient's ribs. Such procedures, commonly referred to as video assisted thorascopic surgery (VATS), aim to reduce patient recovery time by accessing the thoracic cavity through the natural intercostal space without spreading the ribs as in open procedures. This restricted access can sometimes cause problems when removing large specimens. Moreover, in such procedures, e.g. thorascopic wedge resection and lobectomy, it is often necessary to remove a portion of the lung and retrieve it relatively intact for pathology. It is also desirable that the specimen be sufficiently contained to prevent seeding of cancer cells during manipulation and removal.

In designing such specimen retrieval instrumentation, a balance must be struck between the need to provide a retrieval apparatus with a strong enough containment bag to prevent tearing or rupture while providing sufficient rigidity to enable manipulation and removal. Another balance which needs to be achieved is to provide sufficient maneuverability while reducing tissue trauma, e.g., damaging lung tissue, during manipulation and removal. Additionally, the instrumentation on one hand should be able to be inserted through a small access incision or port while on the other hand able to accommodate a wide range of patient sizes and be able to easily remove large specimens and minimize risk of seeding.

Document WO2008/114234 A2 discloses an instrument that forms the basis of the preamble of claim 1.

### SUMMARY

Claim 1 defines the invention and the dependent claims disclose the preferred embodiments, The present disclosure provides a specimen retrieval device. In one aspect, the specimen retrieval device includes an applicator and a specimen retrieval bag disposed within the applicator and deployable therefrom. The specimen retrieval bag includes a closed portion and an open portion. A conduit extends within the specimen retrieval bag and extends along at least a portion of the length thereof to evacuate air from a portion of the retrieval bag.

In some embodiments, the conduit extends from adjacent the open portion to adjacent the closed portion. The conduit has a plurality of perforations to enable communication between an interior of the bag and an interior of the channel.

In some embodiments, the specimen retrieval bag is defined by two generally planar surfaces that are joined together at their edges forming a peripheral edge of the specimen retrieval bag.

In some embodiments, an open cell material such as an open cell foam is positioned within the channel and is substantially compressible and capable of being one of rolled and folded into a compressed state corresponding to when the specimen retrieval bag is in a non-deployed state and within the applicator, to a non-compressed state corresponding to when the specimen retrieval bag is in a deployed state and outside the applicator.

In some embodiments, an upper portion of the specimen retrieval bag includes a generally frustoconical configuration and a lower portion includes a generally rectilinear configuration.

In some embodiments, at least one of the upper portion and lower portion is textured.

In another aspect, the present disclosure provides a specimen retrieval bag configured for use with an applicator, the retrieval bag comprising a first proximal portion and a second distal portion. A conduit having at least one opening extends within the specimen retrieval bag and is configured to evacuate air from the second portion of the specimen retrieval bag.

The conduit can include in some embodiments an open cell material positioned therein. In some embodiments, the open cell material is substantially compressible and capable of being one of rolled and folded into a compressed state corresponding to when the specimen retrieval bag is in a non-deployed state and within the applicator, to a non-compressed state corresponding to when the specimen retrieval bag is in a deployed state and outside the applicator.

In some embodiments, the specimen retrieval bag is defined by two generally planar surfaces joined together at their edges forming a peripheral edge of the specimen retrieval bag.

The specimen retrieval bag can be configured so that the first portion includes a generally frustoconical configuration and the lower portion includes a generally rectilinear configuration.

The upper portion and/or the lower portion can be textured.

The conduit can include a plurality of slots spaced along the conduit to enable communication between an interior of the specimen retrieval bag and an interior of the conduit. The channel can extend at an angle to a longitudinal axis of the specimen retrieval bag.

In another aspect, the present disclosure provides a specimen retrieval device comprising an applicator and a specimen retrieval bag operably disposed within the applicator and deployable therefrom. The specimen retrieval bag defines a longitudinal axis therethrough and includes a closed distal region and an open proximal region. The retrieval bag is defined by two generally planar surfaces joined together at their edges forming a peripheral edge of the specimen retrieval bag. The proximal region of the specimen retrieval bag has a generally frustoconical configuration and the distal region has a generally rectilinear configuration. A perforated channel can be provided extending proximal from the distal region to evacuate air from the distal region.

### BRIEF DESCRIPTION OF THE DRAWINGS

Embodiments of the presently disclosed specimen retrieval apparatus are described hereinbelow with reference to the drawings wherein:
FIG. 1 is a perspective view of a specimen retrieval device in accordance with the present disclosure;
FIG. 1A is a perspective view of an alternate embodiment of the specimen retrieval device of the present disclosure for deploying the surgical retrieval bag;
FIG. 1B is a perspective view of an alternate embodiment of the support for the specimen retrieval bag;
FIG. 2 is an enlarged view of the area of detail depicted in FIG. 1;
FIG. 3A is a schematic view illustrating two sides of a specimen retrieval bag according to another embodiment of the present disclosure;
FIG. 3B is a schematic view illustrating two sides of a specimen retrieval bag according to yet another embodiment of the present disclosure;
FIG. 3C is a perspective view of a specimen retrieval bag according to another alternate embodiment of the present disclosure:
FIG. 3D is an enlarged view of the area of detail of FIG. 3C;
FIGS. 4A-4C are perspective views illustrating various textured surfaces that may line an interior of the specimen retrieval bag depicted in FIGS. 2-3B.

### DETAILED DESCRIPTION OF THE EMBODIMENTS

Embodiments of the present disclosure will now be described in detail with reference to the drawings, in which like reference numerals designate identical or corresponding elements in each of the several views. As used herein, the term distal refers to the portion of the instrument which is further from the user, while the term proximal refers to that portion of the instrument which is closer to the user. In the following description, well-known functions or constructions are not described in detail to avoid obscuring the present disclosure in unnecessary detail.

As used herein with reference to the present disclosure, the term endoscopic refers to instruments having a relatively narrow operating portion for insertion into an access port, a small incision in the skin, or opening in the body. Such procedures are referred to as minimally invasive surgical procedures. Although the presently disclosed specimen retrieval device is described in the context of a minimally invasive thoracic procedure, it is contemplated that the present disclosure may find use in any procedure e.g. laparoscopic procedures, where access to the interior of the body is limited to a relatively small incision or body opening, with or without the use of an access port.

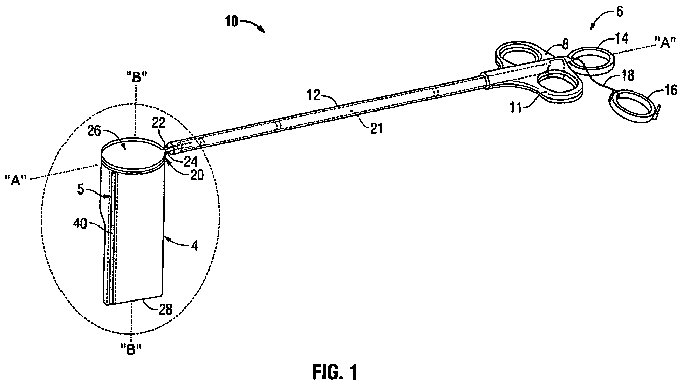

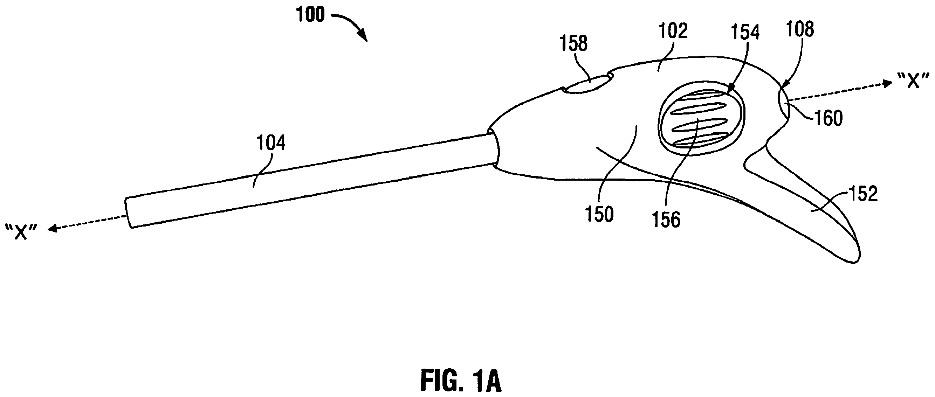

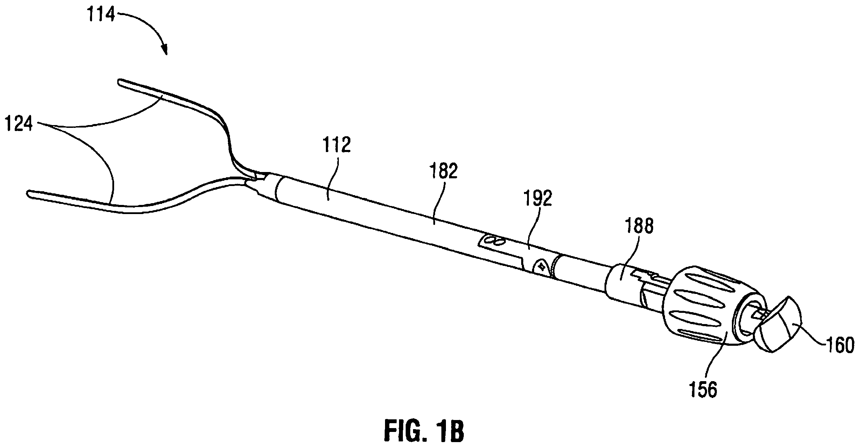

With reference to FIG. 1, a specimen retrieval device 10 including a specimen retrieval bag 4 according to an embodiment of the present disclosure is illustrated.

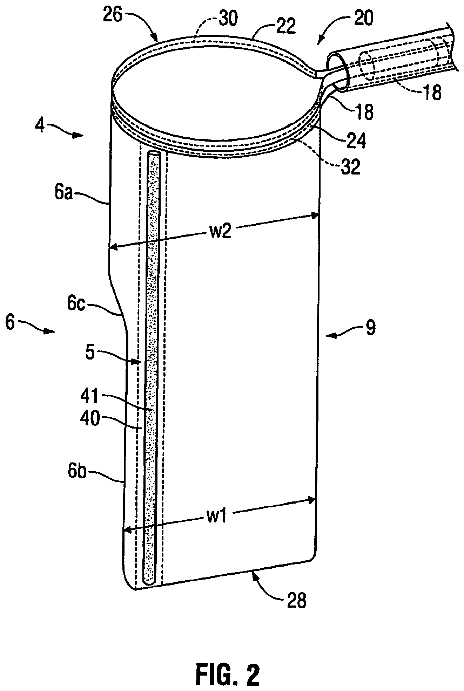

Specimen retrieval device 10 includes a handle assembly 6 that includes handle portions 8 and 11 that are fixedly joined together. An elongated tube or shaft 12 extends from the handle assembly 6 and is dimensioned for insertion through an access port (not shown) for performing minimally invasive surgical procedures. A longitudinal axis "A-A" is defined through the shaft 12 and is oriented in a substantially perpendicular or substantially orthogonal direction with respect to a longitudinal axis "B-B" that is defined through the bag 4 when the bag 4 is in the deployed state (FIG. 1). A slidable drive rod 21 extends within a lumen in the shaft 12 and along a length thereof and operably couples to an actuator that is in the form of a finger loop 14. A distal end of the drive rod 21 is operably coupled to the bag 4. Finger loop 14 is configured for engagement by a user's fingers and is configured to translate or move the drive rod 21 within the shaft 12. A pull ring 16 is operably coupled to a proximal end of a drawstring 18 by any suitable coupling method, e.g., adhesive, and is configured to facilitate pulling the drawstring 18. In the illustrated embodiment, pull ring 16 releasably couples, via one or more suitable coupling methods, e.g., a press or friction fit, to the finger loop 14. A distal end of the drawstring 18 operably couples to a portion, e.g., an open upper portion 26, of the bag 4 (FIG. 2). Drawstring 18 can for example be positioned in a pocket or sleeve extending around a peripheral portion of the bag 4. A deformable spring 20 is operably coupled to the drive rod 21 and includes two generally flexible or resilient strips 22 and 24 that move from a stressed or non-expanded state within shaft 12 to an unstressed or freely expanded state (FIG. 1) when deployed from the shaft 12. In an unstressed or freely expanded condition, the two resilient strips 22 and 24 collectively form a generally circular or "hoop-like" configuration for supporting a periphery of the opening 26 of bag 4. In alternate embodiments, the strips have free ends, such as strips 124 of FIG. 1B at distal region 114. Shaft 112 is movably positioned within an outer sleeve such as a sleeve similar to sleeve 104 of FIG. 1A discussed below. In the initial position, finger loop 14 is in a retracted position, further spaced from handle portions 8 and 11. To deploy the bag 4, finger loop 14 is advanced distally towards handle portions 8 and 11 to the position of FIG. 1, causing the drive rod 21 to be advanced distally, which advances strips 22 and 24 from the confines of shaft 12 to move to the expanded position of FIG. 1 to deploy and open the bag 4. After placement of the tissue specimen within bag 4, drawstring 18 is pulled proximally to close the bag 4 to retain the specimen therein for withdrawal from the body cavity, e.g. thoracic cavity.

For a more detailed description of the specimen retrieval device 10 and operative components associated therewith such as the drawstring, finger loops, drive rod, etc., reference is made to U.S. Patent No. 5,647,372 to Tovey et al.

FIG. 1A illustrates an alternate device for deploying bag 4. Device 100 includes a housing 102, a body portion 150 and a handle portion or gripping portion 152. Body portion 150 includes a proximal aperture 108 that is configured to receive an internal shaft therethrough. Body portion 150 also includes a pair of oval-shaped windows 154 (although other configurations are contemplated). Windows 154 permit the clinician to grasp and manipulate articulation wheel 156 for articulating the retrieval bag relative to longitudinal axis "X-X." Plunger 160 is advanced distally to deploy the retrieval bag from elongated sleeve 104. Surgical retrieval apparatus 10 further includes a release trigger 158 coupled to body portion 150. Release trigger 158 is operable to release plunger 160, which is releasably engageable with the internal shaft. When released, plunger 160 extends proximally from proximal aperture 108 of body portion 150, allowing the clinician to grasp plunger 160 and pull proximally to translate the internal shaft 112 proximally back to the insertion (retracted) position. As the shaft is translated back to the insertion position, an end effector assembly which includes the retrieval bag and supports are translated proximally back within elongated sleeve 104. Further details of these components and their operation are described in provisional patent application 61/430,208, filed January 6, 2011 and published as patent No. US2012179165.

Handle portion 152 of housing 102 extends downwardly and proximally from body portion 150 to define an ergonomically-enhanced configuration for grasping by the clinician. More specifically, the configuration of handle portion 152 permits the clinician to grasp housing 102 in numerous configurations, while still being able to firmly grasp and fully manipulate and operate the device 10. For example, the clinician may grasp housing 102 using a pistol grip, a palm grip, an upside-down grip, a rear grip, a front grip, etc. The specific grip used may depend on the clinician's preference or the surgical procedure being performed.

With reference to FIG. 2, one embodiment of a retrieval bag is shown. Bag 4 is shown for use with device 10 of FIG. 1, but can also be used with other bag deployment devices such as device 100 of FIG. 1A. Bag 4 may be made from any suitable biocompatible materials. Bag 4 (and other bags disclosed herein) can be composed of a flexible film or sheet formed from a substantially transparent polymeric material. The polymeric material is preferably relatively inelastic having a high tensile strength. In one embodiment, the polymeric material is a polyurethane sheet having a thickness that ranges from about 0.001 to about 0.005. In other embodiments, Nylon material can be utilized. Other materials and/or other dimensions are also contemplated. Bag 4 is shown having a generally tubular or elongated configuration that is defined by an openable and closable upper portion or mouth 26 and closed lower portion 28. Bag 4 is proportioned for the purpose of organ or tissue entrapment or removal and is impervious to penetration by cancer cells. The bag 4 (and other bags disclosed herein) can include a coating, such as a polyurethane coating, on the inside and/or outside of the bag to provide on impermeable barrier. A lubricant can also be placed on select portions of the exterior of the bag to facilitate its loading into the shaft during manufacture and facilitate its deployment from the applicator from a folded, rolled or collapsed condition. It is also contemplated that a lubricant can be placed on the exterior surface of the bag, on either portions or on the entire surface, to facilitate its removal from the incision and/or access port extending through the incision.

Upper portion 26 of bag 4 includes a proximal (or upper) circumferential tubular portion or sleeve 30, and a distal (or lower) circumferential tubular portion or sleeve 32, which are spaced apart from each other (each of the sleeves 30 and 32 are shown in phantom in FIG. 2). Proximal and distal sleeves 30 and 32, respectively, are adapted and proportioned to receive the respective spring strips 22, 24 (or strips 124) and drawstring 18, as best seen in FIG. 2. A linear portion (not shown) weakened by perforation, scoring, or the like, extends circumferentially around the mouth 26 of bag 4 and is operably disposed between the proximal and distal sleeves 30 and 32. The scored portion is adapted to tear when the drawstring 18 is pulled with sufficient force so as to close the mouth 26 of the bag 4 distal to the perforation, thereby providing fast detachment of bag 4 from the spring member 20 simultaneously with closure of mouth 26. One skilled in the art will appreciate that alternative methods can also be utilized to detach the bag 4 from the spring member 20, such as by pulling with a grasper or by cutting with a scissors.

Bag 4 has an upper region 6a and lower region 6b, and an intermediate region 6c therebetween. Intermediate region 6c at side 6 tapers distally inwardly so that the transverse dimension or width w1 of lower region 6b is less than the transverse dimension or width w2 of upper region 6a. The opposing side of bag 4 (side 9) in the illustrated embodiment is substantially linear along its length. Thus, the opposing sides of the bag 4 in regions 6a and 6b are substantially linear and substantially parallel.

An elongated pocket is formed on the interior of the bag 4, forming a conduit or channel 40. The conduit 40 can be formed of a material welded to the inside of the bag 4. The material utilized can be the same as the bag or a different material. The conduit 40 extends along a length of the bag 4, illustratively extending from adjacent the open upper portion 26 to adjacent the closed lower portion 28. The conduit 40 has at least one, and preferably a plurality of, perforations along its length, configured to evacuate air from a portion of the bag 4 during removal of the tissue specimen. Note the conduit can alternately extend a shorter length along the bag 4, e.g., terminate more distal of upper portion 26, e.g., spaced further distally from the mouth of the bag so air from the lower portion of the bag travels through the channel and exits into the upper portion of the bag. The conduit 40 is shown formed substantially parallel to the substantially linear sides of the bag but can alternatively be placed at an angle thereto. The angling of the conduit helps the conduit (channel) roll evenly and provides a lower profile as it provides a thinner bundle to more easily fit within the instrument shaft.

In the illustrated embodiment, conduit 40 includes a support such as an open cell material 41, such as open cell foam, positioned therein. Foam 41 may be made from any suitable open cell foam, e.g., foam rubber or polyurethane foam, to enable evacuation of air therethrough. Foam 41 is preferably substantially compressible and capable of being rolled or folded into a compressed state, such as, for example, when the bag 4 is being loaded into the shaft 12 of the device 10 during manufacture. As can be appreciated, having a bag 4 with a compressible foam can facilitate loading the bag 4 into the shaft 12 of the applicator 10, and can maintain the integrity of the channel 40 during use. The foam 41 can also facilitate deployment of the bag 4 by providing some rigidity along the length of the bag 4. It can in some embodiments bias the bag 4 to an open position upon deployment.

In alternate embodiments, instead of a foam or other open cell material, a plurality of longitudinal ribs can be provided in the channel. These ribs would function to prevent occlusion when the tube or channel is flattened or folded.

Foam 41 may be secured to the bag 4 by known securement methods. In one embodiment, the channel 40 is formed by thermoforming an interior surface of the bag 4 into an appropriately sized housing that is configured to maintain the foam 41. Alternatively, a perforated polyurethane sheet or other material can be welded to the interior surface of the bag 4 forming a conduit 40 that is configured to house the foam 41 therein. The conduit 40 serves to constrain the foam 41, which is free to move within the space 5 in conduit 40 as the transverse dimension of the foam is less than a transverse dimension of the conduit 40. The foam 41, as noted above, provides a support to prevent collapse of the conduit 40 to ensure an air passageway is continuously maintained.

In use, a tissue specimen is placed in the bag 4. As the tissue specimen is positioned in the closed bottom portion 28 of the bag 4, the air trapped beneath the tissue specimen is directed past or through the channel 40 during placement as well as when it is being removed. That is, a constant air passage is formed to enable air to continuously seep out of the bottom of the bag and prevent air pocket formation at the bottom of the bag 4. Thus, the configuration of the channel 40 prevents air from becoming trapped beneath the tissue specimen in the closed bottom portion 28. As can be appreciated, this greatly diminishes and/or eliminates the likelihood of enlarging and/or expanding an opening in tissue as the bag 4 is pulled therethrough. It also maintains a longitudinal orientation of the specimen to facilitate removal through the incision. That is, the specimen can be maintained in a sausage-like shape so its long axis is perpendicular to the incision which reduces the force required for removal through the incision or port. Additionally, the evacuation of air prevents the specimen from "balling" at the bottom portion of the bag which can adversely affect removal.

Note bag 4 (and the other bags disclosed herein) can be utilized with the devices 10, 100 disclosed herein or with other deployment devices.

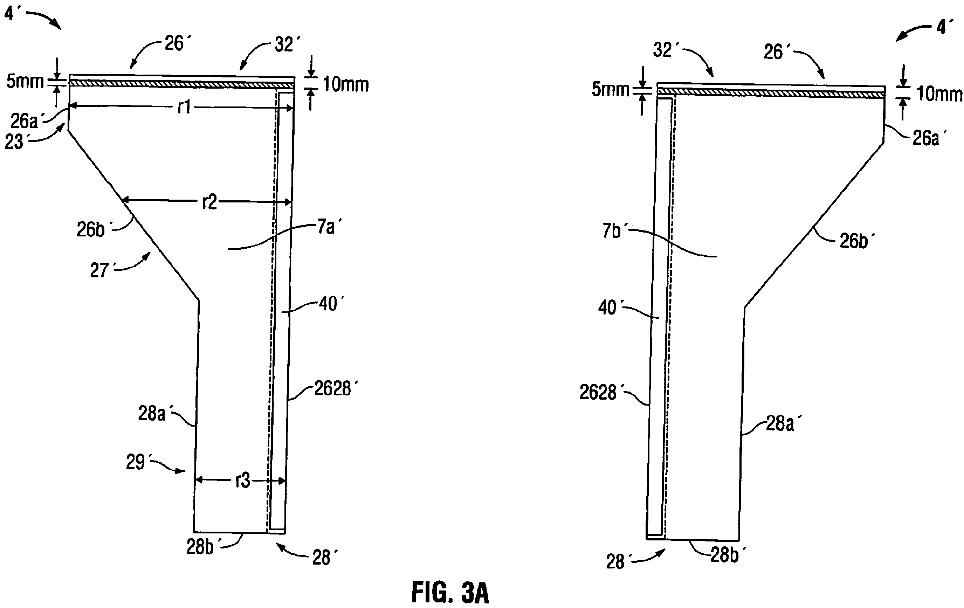

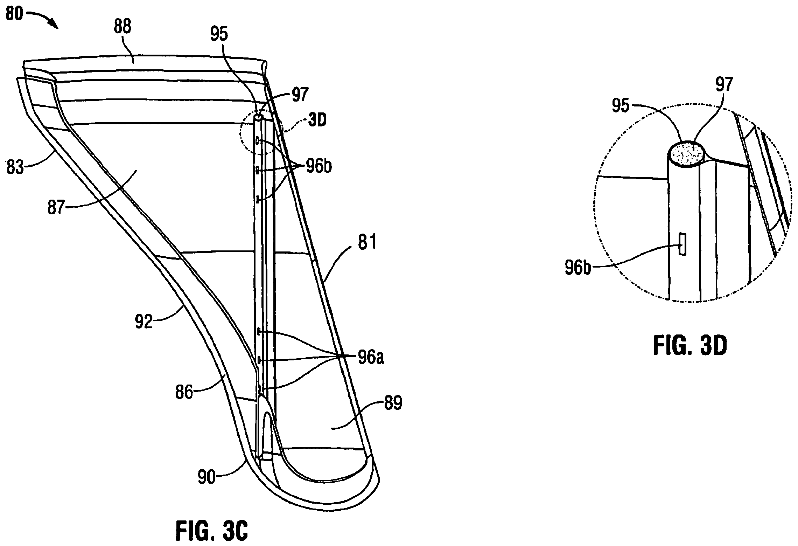

In the alternate embodiment of FIG. 3A bag 4' is defined by two generally planar surfaces 7a' and 7b' that are joined together at their outer peripheral edges forming a substantially continuous outer peripheral edge of the bag 4'. More particularly, and in one particular embodiment, bag 4' is defined by planar surfaces 7a' and 7b' that collectively define an open upper (proximal) portion 26' having edges 26a' and 26b' joined by one or more suitable joining methods, e.g., thermofusing, adhesive, heat curing, etc. Likewise, a closed bottom (distal) portion 28' includes thermofused (or otherwise attached) edges 28a' and 28b'. A connecting thermofused side edge 2628' extends along the length of the bag 4' and connects the open upper portion 26' and closed bottom portion 28', such that the open upper portion 26' forms a generally frustoconical shape at region 27' and the closed bottom portion 28' forms a generally rectilinear shape at region 29'. Uppermost region 23' forms a generally rectilinear shape. Thus, region 23' has a first transverse dimension or width r1, region 27' has a progressive taper, tapering distally to second smaller transverse dimensions e.g. width r2, and region 29' has third smaller transverse dimension or width r3. As can be appreciated, in the illustrated embodiment, width r1 and width r3 are substantially constant, with r1 exceeding r3.

The dimensions of the edges 26a', 26b', 28a', 28b', and 2628' may be varied depending on a specific surgical procedure, specific opening size of the bag 4', type of tissue being excised, a manufacturer's preference, etc. In one particular embodiment, the bag 4' may include an opening 26' that ranges from about 5.11 inches (130 mm) to about 6.29 inches (160 mm), an edge 28a' that ranges from about 3.93 inches (100 mm) to about 7.84 inches (200 mm), an edge 28b' that ranges from about 1.96 inches (50 mm) to about 3.14 inches (80 mm), and an edge 2628' that ranges from about 9.84 inches (250 mm) to about 15.15 inches (385 mm). Other dimensions are also contemplated.

A support, which can be in the form of an open cell material, such as an open cell foam as described above, in conjunction with bag 4 is positioned in a channel or conduit 40' and extends along an interior of the bag 4', the foam and channel functioning identically to foam 41 and channel 40 of FIG. 2. In the embodiment illustrated in FIG. 3A, the channel 40' is illustrated extending along the interior of the bag 4' adjacent the edge 2628'. For illustrative purposes, the channel 40' is shown on each of the planer surfaces 7a' and 7b'. As can be appreciated, the channel 40' may be secured to an interior of the bag 4' via the methods described above with respect to bag 4 or by other methods. The channel 40' is shown substantially parallel to substantially linear edge 2628', but could alternatively be placed at an angle to the longitudinal axis of the bag. Also, although shown as extending the length of the bag, a shorter length channel could also be provided, e.g. the channel can terminate distal of the bag opening. Multiple channels in this and the other bags disclosed herein are also contemplated for air evacuation.

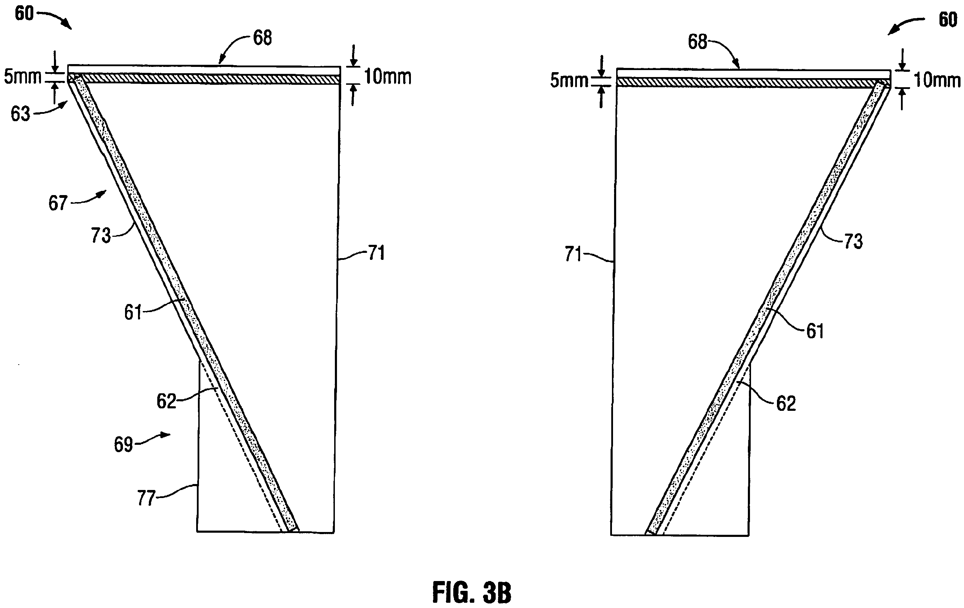

A channel 32' configured to function similar to that of sleeve 32 is formed around the top of the bag 4'. Channel 32' is formed by folding over a top edge of the bag 4' into the bag opening 26'. The top edge is attached to the interior of the bag 4' by using a weld that in one embodiment is approximately 0.196 inches (5 mm) wide. In the embodiment illustrated in FIGS. 3A and 3B, the width of the channel 32' is approximately 0.393 inches (10 mm) wide. Other dimensions are also contemplated.

In the alternate embodiment of FIG. 3B, specimen retrieval bag 60 includes a channel 62 extending along the inner wall. The channel or conduit 62 can be formed as integral with the bag material or alternatively can be in the form of a separate tube or material attached to the bag 60, e.g., attached to an inner surface in a manner discussed above with respect to channels 40, 40'. The channel 62 includes at least one opening or slot along its length to allow the passage of air into the channel 62. Preferably, a plurality of slots or openings are provided to enable communication between the air and/or fluid in the bag 60 and the interior of the channel 62. The channel 62 (and channels 40, 40' and 95) in some embodiments can also terminate at its distal end spaced from the bottom of the bag 60 to communicate at a distal opening with the interior of the bag to provide another path for the escape of air fluid. Proximal end 63 of the channel 62 is open to communicate with the exterior of the bag. Other length channels are also contemplated, e.g., the proximal opening of channel 60 can be more distal of the mouth of the bag so air exits into the proximal region of the bag.

Positioned within the channel 62 is a support member (or support members) 61 configured for preventing collapse of the channel 62. It can also in some embodiments be utilized to bias the specimen retrieval bag 60 in an open position upon deployment from specimen retrieval apparatus, i.e., upon translation of the end effector assembly from the retracted position to the extended position. Support member 61 may be formed from, for example, an open cell material, such as open cell foam, or other suitable material. The open cell material, e.g., foam, enables the passage of air and/or fluid therethrough. The open cell material is preferably of a transverse cross-section less than the transverse cross-section of the channel 62 for ease of manufacture. Also in this manner, air and/or fluid entering the channel 62 from the bag 60 can flow around the open cell material through the channel 62. Note that due to the open cell material, the air or fluid can also flow through the open cell foam itself. Therefore, alternatively, the open cell material can have a transverse cross section substantially equal to the transverse cross-section of the channel. This open cell support ensures that if channel 62 collapses or is compressed during specimen retrieval, air and fluid can still escape. The open cell material, e.g., foam, and channel of the other bag embodiments disclosed herein also function in the same fashion and can be configured in the same fashion. Various configurations of the open cell material and channel discussed herein are applicable to each of the bag embodiments. The escape of air and fluid is caused as the pressure is applied to the bag 60 during withdrawal through an access port or body opening. As the bag 60 is compressed, the air and/or fluid is forced proximally through the channel 62, exiting the open proximal end 63 and/or through slots in the bag 60 at a proximal region. Thus, this decrease in pressure prevents balling of the specimen at the bottom of the bag 60 and facilitates removal.

In the embodiment of FIG. 3B, the bag 60 has proximal region 67 with a mouth or open end 68 and a distal region 69. Distal region 69 has a smaller transverse dimension than proximal region 67. In the illustrated embodiment, bag 60 has a first side 71 and an angled side 73 in the proximal region 67 opposite the first side 71. This side 73 tapers inwardly distally such that the transverse dimension of proximal region 67 progressively decreases toward the distal region 69. Wall 77 opposite the wall 71 in the distal region 69 extends substantially parallel to the wall 71. Thus, as can be appreciated, the transverse dimension remains substantially the same in the distal region 69, but is smaller than the transverse dimension in the proximal region 67. Thus, a substantially frustoconical portion is formed in upper (proximal) region 67 and a substantially rectilinear portion is formed at the lower (distal) region 69. Other shaped bags are also contemplated.

In the alternate embodiment of FIG. 3C, bag 80 has a proximal region 87 with a mouth or open end 88 and a distal region 89. Distal region 89 has a smaller transverse dimension than proximal region 87. In the illustrated embodiment, bag 80 has a first side 81 and an angled side 83 in the proximal region 81 opposite the first side 81. This side 83 has an angled wall transitioning into a curved wall 92; each tapering inwardly distally such that the transverse dimension of proximal region 87 progressively decreases toward the distal region 89. Wall 86 opposite the wall 81 in the distal region 89 transitions to a curved lower wall 90. Thus, as can be appreciated, the transverse dimension remains substantially the same in the distal region 89, until the curved lower wall 90 where the transverse dimension decreases distally.

A channel 95 is angled with respect to the longitudinal axis as shown and has lower slots 96a and upper slots 96b along its length communicating with the interior for passage of air. Other slot configurations and, spacings and a different number of slots are also contemplated. A support 97 in the form of an open cell material such as open cell foam functions in the same manner as described above. Open cell support 97 can have a transverse dimension less than a transverse dimension of the channel 95 to provide a gap for air passage. The length of channel 95 can be shorter or longer than shown in FIG. 3C.

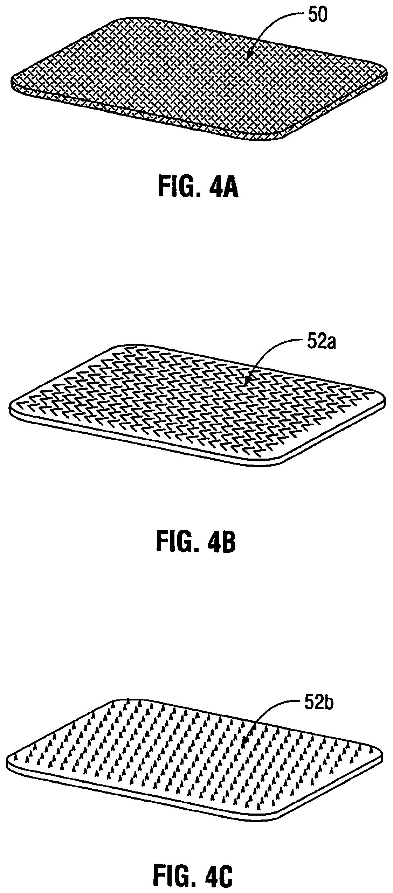

In certain embodiments, see FIGS. 4A-4C for example, an internal surface of the bags 4, 4', 68, and 87 may be textured. More particularly, an internal surface of the open upper portions 26, 26' and 68 may include one or more various textured patterns. The textured patterns are configured to increase a friction coefficient associated with the internal surface of the open upper portions 26, 26', 68, and 88. For example, in FIG. 4A, the textured pattern includes a plurality of spaced-apart projections 50. FIG. 4B illustrates the plurality of spaced-apart projections 50 as having a chevron configuration 52a. FIG. 4C illustrates the plurality of spaced-apart projections 50 as having a pyramidal configuration 52b. As can be appreciated, different configurations of the plurality of spaced-apart projections may be utilized to achieve the purposes described herein. The texture can be provided on a portion of the internal surface or on the entire internal surface of the bags.

From the foregoing and with reference to the various figure drawings, those skilled in the art will appreciate that certain modifications can also be made to the present disclosure without departing from the scope of the claims. For example, instead of the plurality of spaced-apart projections 50, or in combination therewith, an interior surface of the open upper portions may be coated with a "sticky" material to facilitate "gripping" or "pulling" a tissue specimen.

While several embodiments of the invention have been shown in the drawings, it is not intended that the scope of the invention be limited thereto. Therefore, the above description should not be construed as limiting, but merely as exemplifications of particular embodiments. Those skilled in the art will envision other modifications within the scope of the claims appended hereto.

## Claims

1. A specimen retrieval bag (4, 4', 60, 80) configured for use with an applicator (10, 100), the specimen retrieval bag comprising a closed portion (28, 28', 69, 89) and an open portion (26, 26', 68, 88) configured to be closed by a drawstring (18), and **characterised by** a conduit (40, 40', 62, 95) extending within the specimen retrieval bag and extending along at least a portion of the length of the specimen retrieval bag wherein the conduit has a plurality of perforations along its length configured to enable communication between an interior of the bag and an interior (5) of the conduit to evacuate air from a portion of the specimen retrieval bag.

2. The specimen retrieval bag (4, 4', 60, 80) according to claim 1, wherein the conduit (40, 40', 62, 95) extends from adjacent the open portion (26, 26', 68, 88) to adjacent the closed portion (28, 28', 69, 89).

3. The specimen retrieval bag (4, 4', 60, 80) according to claims 1 or 2, wherein the conduit (40, 40', 62, 95) contains an open cell material (41, 61, 97) positioned therein.

4. The specimen retrieval bag (4, 4', 60, 80) according to any of claims 1-3, wherein an elongated pocket formed on the interior of the bag forms the conduit (40, 40', 62, 95).

5. The specimen retrieval bag (4, 4', 60, 80) according to any preceding claim, wherein the specimen retrieval bag is defined by two generally planar surfaces (7a', 7b') joined together at their edges (26a', 26b', 28a', 28b', 2628') forming a peripheral edge of the specimen retrieval bag.

6. The specimen retrieval bag (4, 4', 60, 80) according to claim 3, wherein the open cell material (41, 61, 97) is substantially compressible and capable of being one of rolled and folded into a compressed state corresponding to when the specimen retrieval bag is in a non-deployed state and within an applicator (10, 100), to a non-compressed state corresponding to when the specimen retrieval bag is in a deployed state and outside the applicator.

7. The specimen retrieval bag (4, 4', 60, 80) according to claim 3 or claim 6, wherein the open cell material (41, 61, 97) is open cell foam.

8. The specimen retrieval bag (4, 4', 60, 80) according to any preceding claim, wherein the at least one of the open upper portion (26, 26', 68, 88) and the closed lower portion (28, 28', 69, 89) of the bag is textured.

9. The specimen retrieval bag (4, 4', 60, 80) according to any of preceding claim in combination with an applicator (10, 100), the bag disposed within the applicator and deployable therefrom, wherein the applicator includes a handle assembly (6, 150) having an actuator (14, 160), and the specimen retrieval bag is deployable from the applicator when the actuator is moved distally.

10. The specimen retrieval bag (4, 4', 60, 80) of any preceding claim, wherein the conduit (40, 40', 62, 95) extending within the specimen retrieval bag terminates distally from the open upper portion (26, 26', 68, 88) to evacuate air from the closed lower portion (28, 28', 69, 89) of the bag.

11. The specimen retrieval bag (4, 4', 60, 80) according to claim 10, wherein the air is evacuated from the closed lower portion (28, 28', 69, 89) and exits into the open upper portion (26, 26', 68, 88) of the bag.

12. The specimen retrieval bag (4, 4', 60, 80) according to any preceding claim, wherein the conduit (40, 40', 62, 95) extends at an angle to a longitudinal axis of the specimen retrieval bag.

13. The specimen retrieval bag (4, 4', 60, 80) according to any preceding claim, wherein the perforations are configured as a plurality of slots (96a, 96b) spaced along the conduit (40, 40', 62, 95).

14. The specimen retrieval bag (4, 4', 60, 80) of any preceding claim, wherein the open upper portion (26, 26', 68, 88) has a generally frustoconical configuration tapering inwardly toward the closed lower portion (28, 28', 69, 89), and the closed lower portion has a generally rectilinear configuration.

## Patentansprüche

1. Probenentnahmebeutel (4, 4', 60, 80), der zur Verwendung mit einem Applikator (10, 100) konfiguriert ist, wobei der Probenentnahmebeutel einen geschlossenen Abschnitt (28, 28', 69, 89) und einen offenen Abschnitt (26, 26', 68, 88), der zum Verschließen durch eine Zugschnur (18) konfiguriert ist, umfasst und **gekennzeichnet dadurch, dass** ein Rohr (40, 40', 62, 95) sich innerhalb des Probenentnahmebeutels erstreckt und sich entlang wenigstens eines Abschnitts der Länge des Probenentnahmebeutels erstreckt, wobei das Rohr mehrere Perforationen entlang einer Länge davon aufweist, die dafür konfiguriert sind, eine Kommunikation zwischen einem Innenraum des Beutels und einem Innenraum (5) des Rohres zu ermöglichen, um Luft aus einem Abschnitt des Probenentnahmebeutels zu evakuieren.

2. Probenentnahmebeutel (4, 4', 60, 80) nach Anspruch 1, wobei sich das Rohr (40, 40', 62, 95) angrenzend von dem offenen Abschnitt (26, 26', 68, 88) zu dem angrenzenden geschlossenen Abschnitt (28, 28', 69, 89) erstreckt.

3. Probenentnahmebeutel (4, 4', 60, 80) nach Anspruch 1 oder 2, wobei das Rohr (40, 40', 62, 95) ein offenzelliges Material (41, 61, 97) enthält, das darin positioniert ist.

4. Probenentnahmebeutel (4, 4', 60, 80) nach einem der Ansprüche 1 bis 3, wobei eine in dem Innenraum des Beutels ausgebildete längliche Tasche das Rohr (40, 40', 62, 95) ausbildet.

5. Probenentnahmebeutel (4, 4', 60, 80) nach einem der vorhergehenden Ansprüche, wobei der Probenentnahmebeutel durch zwei allgemein planare Oberflächen (7a', 7b'), die an ihren Kanten (26a', 26b', 28a', 28b', 2628') miteinander verbunden sind, die eine Umfangskante des Probenentnahmebeutels ausbilden, definiert ist.

6. Probenentnahmebeutel (4, 4', 60, 80) nach Anspruch 3, wobei das offenzellige Material (41, 61, 97) im Wesentlichen komprimierbar ist und in einen komprimierten Zustand gerollt und gefaltet werden kann, der dem entspricht, wenn der Probenentnahmebeutel in einem nicht entfalteten Zustand und innerhalb eines Applikators (10, 100) ist, in einem nicht komprimierten Zustand, der dem entspricht, wenn der Probenentnahmebeutel in einem entfalteten Zustand und außerhalb des Applikators ist.

7. Probenentnahmebeutel (4, 4', 60, 80) nach Anspruch 3 oder 6, wobei das offenzellige Material (41, 61, 97) offenzelliger Schaum ist.

8. Probenentnahmebeutel (4, 4', 60, 80) nach einem der vorhergehenden Ansprüche, wobei der offene obere Abschnitt (26, 26', 68, 88) und/oder der geschlossene untere Abschnitt (28, 28', 69, 89) des Beutels texturiert ist.

9. Probenentnahmebeutel (4, 4', 60, 80) nach einem der vorhergehenden Ansprüche in Kombination mit einem Applikator (10, 100), wobei der Beutel innerhalb des Applikators angeordnet und von diesem entfaltbar ist, wobei der Applikator eine Griffanordnung (6, 150) mit einem Aktuator (14, 160) enthält, und der Probenentnahmebeutel von dem Applikator entfaltbar ist, wenn der Aktuator distal bewegt wird.

10. Probenentnahmebeutel (4, 4', 60, 80) nach einem der vorhergehenden Ansprüche, wobei das Rohr (40, 40', 62, 95), das sich in dem Probenentnahmebeutel erstreckt, distal von dem offenen oberen Abschnitt (26, 26', 68, 88) endet, um Luft aus dem geschlossenen unteren Abschnitt (28, 28', 69, 89) des Beutels zu evakuieren.

11. Probenentnahmebeutel (4, 4', 60, 80) nach Anspruch 10, wobei die Luft aus dem geschlossenen unteren Abschnitt (28, 28', 69, 89) evakuiert wird und in den offenen oberen Abschnitt (26, 26', 68, 88) des Beutels austretet.

12. Probenentnahmebeutel (4, 4', 60, 80) nach einem der vorhergehenden Ansprüche, wobei sich das Rohr (40, 40', 62, 95) in einem Winkel zu einer Längsachse des Probenentnahmebeutels erstreckt.

13. Probenentnahmebeutel (4, 4', 60, 80) nach einem der vorhergehenden Ansprüche, wobei die Perforationen als mehrere Schlitze (96a, 96b) konfiguriert sind, die entlang des Rohrs (40, 40', 62, 95) beabstandet sind.

14. Probenentnahmebeutel (4, 4', 60, 80) nach einem der vorhergehenden Ansprüche, wobei der offene obere Abschnitt (26, 26', 68, 88) eine im Allgemeinen kegelstumpfförmige Konfiguration aufweist, die nach innen zum geschlossenen unteren Abschnitt (28, 28', 69, 89) hin konisch zuläuft und der geschlossene untere Abschnitt eine allgemein geradlinige Konfiguration aufweist.

## Revendications

1. Sac de récupération d'échantillon (4, 4', 60, 80) conçu pour une utilisation avec un applicateur (10, 100), le sac de récupération d'échantillon comprenant une partie fermée (28, 28', 69, 89) et une partie ouverte (26, 26', 68, 88) conçue pour être fermée par un cordon (18), et **caractérisée par** un conduit (40, 40', 62, 95) s'étendant dans le sac de récupération d'échantillon et s'étendant le long d'au moins une partie de la longueur du sac de récupération d'échantillon, le conduit comportant une pluralité de perforations sur sa longueur conçues pour permettre la communication entre l'intérieur du sac et l'intérieur (5) du conduit afin d'évacuer l'air d'une partie du sac de récupération d'échantillon.

2. Sac de récupération d'échantillon (4, 4', 60, 80) selon la revendication 1, dans lequel le conduit (40, 40', 62, 95) s'étend de la partie ouverte (26, 26', 68, 88) à la partie fermée de manière adjacente (28, 28', 69, 89).

3. Sac de récupération d'échantillon (4, 4', 60, 80) selon la revendication 1 ou 2, dans lequel le conduit (40, 40', 62, 95) contient un matériau à cellules ouvertes (41, 61, 97) placé dans celui-ci.

4. Sac de récupération d'échantillon (4, 4', 60, 80) selon l'une quelconque des revendications 1 à 3, dans lequel une poche allongée formée à l'intérieur du sac forme le conduit (40, 40', 62, 95).

5. Sac de récupération d'échantillon (4, 4', 60, 80) selon l'une quelconque des revendications précédentes, dans lequel le sac de récupération d'échantillon est défini par deux surfaces généralement planes (7a', 7b') assemblées l'une à l'autre sur leurs bords (26a', 26b', 28a', 28b', 2628') formant un bord périphérique du sac de récupération d'échantillon.

6. Sac de récupération d'échantillon (4, 4', 60, 80) selon la revendication 3, dans lequel le matériau à cellules ouvertes (41, 61, 97) est sensiblement compressible et peut être roulé ou plié dans un état comprimé correspondant au moment où le sac de récupération d'échantillon est dans un état non déployé et à l'intérieur d'un applicateur (10, 100), à un état non comprimé correspondant au moment où le sac de récupération d'échantillon est dans un état déployé et à l'extérieur de l'applicateur.

7. Sac de récupération d'échantillon (4, 4', 60, 80) selon la revendication 3 ou 6, dans lequel le matériau à cellules ouvertes (41, 61, 97) est une mousse à cellules ouvertes.

8. Sac de récupération d'échantillon (4, 4', 60, 80) selon l'une quelconque des revendications précédentes, dans lequel l'au moins une des parties supérieure ouverte (26, 26', 68, 88) et inférieure fermée (28, 28', 69, 89) du sac est texturée.

9. Sac de récupération d'échantillon (4, 4', 60, 80) selon l'une quelconque des revendications précédentes, en combinaison avec un applicateur (10, 100), le sac étant disposé à l'intérieur de l'applicateur et pouvant être déployé à partir de celui-ci, l'applicateur comprenant un ensemble poignée (6, 150) comportant un actionneur (14, 160) et le sac de récupération d'échantillon pouvant être déployé à partir de l'applicateur lorsque l'actionneur est déplacé de manière distale.

10. Sac de récupération d'échantillon (4, 4', 60, 80) selon l'une quelconque des revendications précédentes, dans lequel le conduit (40, 40', 62, 95) s'étendant dans le sac de récupération d'échantillon se termine de manière distale à partir de la partie supérieure ouverte (26, 26', 68, 88) pour évacuer l'air de la partie inférieure fermée (28, 28', 69, 89) du sac.

11. Sac de récupération d'échantillon (4, 4', 60, 80) selon la revendication 10, dans lequel l'air est évacué de la partie inférieure fermée (28, 28', 69, 89) et sort par la partie supérieure ouverte (26, 26', 68, 88) du sac.

12. Sac de récupération d'échantillon (4, 4', 60, 80) selon l'une quelconque des revendications précédentes, dans lequel le conduit (40, 40', 62, 95) s'étend à un angle par rapport à un axe longitudinal du sac de récupération d'échantillon.

13. Sac de récupération d'échantillon (4, 4', 60, 80) selon l'une quelconque des revendications précédentes, dans lequel les perforations sont conçues comme une pluralité de fentes (96a, 96b) espacées le long du conduit (40, 40', 62, 95).

14. Sac de récupération d'échantillon (4, 4', 60, 80) selon l'une quelconque des revendications précédentes, dans laquelle la partie supérieure ouverte (26, 26', 68, 88) a une configuration généralement tronconique s'effilant vers l'intérieur en direction de la partie inférieure fermée (28, 28', 69, 89), et la partie inférieure fermée a une configuration généralement rectiligne.
